# FASCICULE DE BREVET EUROPEEN

(11) **EP 2 459 150 B1**
(45) Date de publication et mention de la délivrance du brevet: **19.06.2019**
(21) Numéro de dépôt: 10752058.7
(22) Date de dépôt: 20.07.2010
(51) Int. Cl.: A61K 6/083

(54) **COMPOSITION POUR BARRIERE DENTAIRE COMPRENANT AU MOINS UN INDICATEUR PERMETTANT LE SUIVI DE LA REACTION DE POLYMERISATION**
ZAHNBARRIEREZUSAMMENSETZUNG MIT MINDESTENS EINEM INDIKATOR ZUR NACHVERFOLGUNG DER POLYMERISATIONSREAKTION
DENTAL BARRIER COMPOSITION INCLUDING AT LEAST ONE INDICATOR ENABLING THE FOLLOW-UP OF THE POLYMERIZATION REACTION

(30) Priorité: 30.07.2009 FR 0955372
(43) Date de publication de la demande: 06.06.2012
(73) Titulaire: Produits Dentaires Pierre Rolland, 33700 Merignac (FR)
(72) Inventeur: MAURAT, Vincent, 33160 Saint-Médard en Jalles (FR); FOURNIE, Marguerite, F-33510 Andernos-les-bains (FR)
(74) Mandataire: Nevant, Marc
(86) Numéro de dépôt international: PCT/FR2010/051532
(87) Numéro de publication internationale: WO 2011/012796

(56) Documents cités:
- EP-A1- 0 443 269
- EP-A2- 1 734 057
- WO-A1-2005/074863
- WO-A1-2008/096182
- WO-A1-2009/067236
- US-A- 2 092 549
- US-A- 6 086 370
- US-A1- 2004 161 724
- US-B1- 6 444 725

## Description

La présente invention porte sur une nouvelle composition pour barrière dentaire comprenant au moins un monomère, au moins un système initiateur de polymérisation, et au moins un indicateur permettant le suivi de la réaction de polymérisation, ainsi que l'utilisation d'un tel indicateur coloré dans le suivi de la réaction de polymérisation lors de la réalisation de barrières dentaires.

Les compositions selon l'invention sont destinées à être utilisées par un praticien dentaire qualifié sur ses patients, lors de toute opération nécessitant la protection d'une zone de la bouche.

### ETAT DE LA TECHNIQUE

Les barrières dentaires, également appelées digues dentaires existent depuis de nombreuses années sous différentes formes.

Le brevet US 2 092 549 décrit une digue dentaire pré-moulée réalisée en latex, que l'on pose sur la zone à protéger. Ces digues présentent l'inconvénient de ne pas adhérer à la zone à protéger, et ainsi de ne pas rester bien en place pendant les opérations dentaires à réaliser.

Le brevet US 7 157 502 au nom de Pulpdent, décrit une méthode pour former une barrière dentaire polymérisable, consistant à faire polymériser un polymère de haut poids moléculaire sur le tissu dentaire du patient. On note que dans ce document les compositions comprennent des polymères et non des monomères comme produit de départ, afin de minimiser la chaleur dégagée par la polymérisation, et ainsi de réduire la douleur chez le patient.

Le brevet EP 0 991 368 au nom d'Ultradent, divulgue des compositions photo-polymérisables pour faire une barrière dentaire, comprenant au moins un monomère, au moins un agent durcissant, et au moins un réducteur organique de la force de et/ou un agent accentuant l'adhérence au tissu et/ou un matériau réfléchissant. Cette composition ne permet donc pas de suivre la polymérisation de la barrière dentaire qui est réalisée, ce qui est peu commode lors de l'utilisation de la composition en pratique.

La demande WO 2009/067236 décrit une barrière dentaire comprenant un composé acrylique polymérisable, un système de polymérisation et éventuellement un indicateur coloré du durcissement et des charges particulaires. Le brevet US 6 444 725 décrit des mastics dentaires comprenant notamment une résine durcissable et un colorant. La demande WO 2008/096182 décrit un matériau pour protéger les gommes qui comprend au moins une substance anti-oxydante et/ou une substance anti-inflammatoire, sédative ou anti-irritante.

Toutefois, la demanderesse a cherché une nouvelle composition pour faire des barrières dentaires, de préférence par photochimie, permettant de résoudre les problèmes soulevés dans les compositions de l'art antérieur existantes.

En effet, du fait de la chaleur générée pendant la polymérisation d'une digue dentaire photopolymérisable, les dentistes ont souvent pour habitude de limiter la gêne des patients en polymérisant le matériau par intervalles d'une ou deux secondes. Or, ces matériaux doivent être polymérisés pendant 10 à 20 secondes par l'intermédiaire d'une lumière intense de longueur d'onde appropriée. Le risque est donc de laisser certaines zones non polymérisés et donc de risquer l'ingestion par le patient, de dérivés méthacrylates toxiques non piégés par la réaction de polymérisation, ou lors de certaines utilisations, par exemple relevant du domaine du blanchiment, de brûler voire nécroser les zones non polymérisées. De plus, il est nécessaire d'isoler correctement la zone traitée par une polymérisation parfaite, les produits appliqués par la suite pouvant générer des agressions chimiques ou thermiques sur les zones non correctement protégées. Plus généralement, les protections polymérisables sont également utilisées pour isoler une zone de traitement lors d'un traitement dentaire.

### BUTS DE L'INVENTION

Ainsi, la présente invention a pour but principal de fournir une nouvelle composition photopolymérisable, pour faire une barrière dentaire, permettant d'isoler les tissus dentaires, dont l'état de polymérisation peut être contrôlé visuellement par l'utilisateur, ce qui permet à l'utilisateur de bien faire en sorte que la polymérisation soit complète sur l'ensemble de la barrière dentaire formée, et ainsi d'obtenir une protection optimale de l'ensemble des tissus à protéger.

La présente invention a également pour but principal de fournir une composition photopolymérisable qui comprend comme composés de base des monomères, et non des composés déjà polymérisés, ce qui permet d'obtenir un procédé de fabrication de la composition plus simple et moins coûteux, utilisable à l'échelle industrielle.

La présente invention a enfin également pour but de fournir une composition photopolymérisable qui, par sa composition et le suivi de sa polymérisation, permet d'éviter toute sensation de brûlure ou d'inconfort inhérente à la réaction de polymérisation qui produit *in situ* de la chaleur.

### DESCRIPTION DE L'INVENTION

Un premier objet de la présente invention porte sur une nouvelle composition précurseur pour barrière dentaire, comprenant un mélange de monomères choisis parmi le diuréthane diméthacrylate, le triéthylène glycol diméthacrylate, le bisphénol A diméthacrylate, et le méthacrylate de méthyle, au moins un épaississant, un moyen d'amorçage photochimique de polymérisation, et au moins un indicateur coloré pour suivre la réaction de polymérisation.

Cette composition constitue ainsi un précurseur permettant l'obtention d'une barrière dentaire après photopolymérisation de ladite composition.

La composition selon l'invention comprend :
- 50 à 90 % en poids de ladite composition du mélange de monomères,
- 10 à 49 % en poids de ladite composition d'au moins un épaississant,
- jusqu'à 2 % en poids de ladite composition du moyen d'amorçage photochimique de polymérisation, et
- jusqu'à 5 % en poids de ladite composition d'au moins un indicateur coloré pour suivre la réaction de polymérisation.

De manière préférée, le mélange de monomères et constitué du diuréthane diméthacrylate et du triéthylène glycol diméthacrylate.

Dans la composition selon l'invention, d'autre part, ledit au moins un épaississant peut être choisi parmi l'alcool cétylique, la silice colloïdale anhydre, le dibéhénate de glycérol, l'acide alginique, l'alginate de sodium, l'alginate de potassium, l'alginate d'ammonium, l'alginate de calcium, l'alginate de propane-1,2-diol, l'agar, la carragénanne, le furcelleran, la gomme de caroube, la gomme d'avoine, la gomme de Guar, le tragacanthe, la gomme arabique, la gomme xanthane, la gomme karaya, la gomme gellane, le sorbitol, le mannitol, le glycerol, le stéarate de polyoxyéthylène, le stéarate de polyoxyéthylène, le monolaurate de polyoxyéthylène-20-sorbitan, le monooléate de polyoxyéthylène-20-sorbitan, le monopalmitate de polyoxyéthylène-20-sorbitan, le monostearate de polyoxyéthylène-20-sorbitan, le tristéarate de polyoxyéthylène-20-sorbitan, la pectine, la gélatine, la cellulose, la cellulose de méthyle, la cellulose d'éthyle, la cellulose d'hydroxypropyle, la cellulose d'hydroxypropylméthyle, la cellulose de méthyléthyle, la cellulose de carboxyméthyl, les sels d'acide gras, les acides gras mono- and di-glycérides, les esters de mono- and di-glycérides, les sucre d'esters d'acides gras, les sucre glycérides, les esters polyglycérol d'acides gras, le polyricinoléate polyglycérol, les esters propylèneglycol d'acides gras, les mélange d'esters glycérol- and propylèneglycol d'acide lactique et d'acides gras, l'huile de soja estérifiée, le stéarate de sorbitane, le tristéarate de sorbitane, le monolaurate de sorbitane, le monooléate de sorbitane, le monopalmitate de sorbitane et leurs mélanges.

De manière préférée, ledit épaississant peut être choisi parmi l'alcool cétylique, la silice colloïdale anhydre, le dibéhénate de glycérol, et leurs mélanges.

En particulier, selon l'invention, ledit épaississant peut être choisi parmi les produits commerciaux constitués essentiellement de l'un ou plusieurs des produits cités ci-dessus. Par exemple, l'Aérosil 200® qui est essentiellement constitué de silice colloïdale anhydre, peut être utilisé en tant que silice colloïdale anhydre.

Selon l'invention, la composition adhésive photosensible comporte un moyen d'amorçage photochimique dont la composition peut être variée. Les moyens d'amorçage peuvent être multiples et on parlera alors d'un système d'amorçage multicomposant qui peut donner lieu à une polymérisation ou réticulation en chaine hybride ou duale.

Les exemples donnés ci-après ne sont en aucun cas limitatifs de l'invention et doivent permettre au formulateur de mieux faire son choix parmi les composés à sa disposition, notamment en fonction de l'utilisation souhaitée de la composition selon l'invention.

Les moyens d'amorçage peuvent être constitués d'au moins un photoamorçeur apte à amorcer un mécanisme de polymérisation sous l'effet d'un rayonnement de réticulation de longueur d'onde À. Dans le présent texte λ désigne aussi bien une radiation de longueur d'onde unique qu'un domaine de longueur d'onde centré sur la valeur mentionnée. Par ailleurs l'expression « rayonnement de réticulation » désigne un rayonnement électromagnétique apte à stimuler la génération de radicaux libres.

Selon un premier aspect des moyens d'amorçage, ceux-ci sont des moyens de photoamorçage.

Selon une première variante de cet aspect, le photoamorçeur est du type apte à générer des radicaux libres via un mécanisme de photoclivage homolytique. Les photoamorçeurs donnant lieu à de tels processus appartiennent à diverses familles et les photoamorçeurs selon l'invention peuvent être choisi parmi le dialkylcétal de benzyle, l'éther de benzoine, l'α-hydroxy, α-alkyl phénylcétone, le benzoyle de cyclohexanol, les oxydes de phosphine de triméthylbenzoyle et plus généralement les bis-acyl- d'oxyde de phosphine, l'α-amino thioalkylphénylphénylcétone, l'α-amino morpholino-phénylcétone, les esters sulfoniques de l'α-hydroxy méthylbenzoïne, et leurs dérivés. Il existe également de nombreux autres amorceurs photoclivables dans lesquels la génération de radicaux se fait à la suite d'une série de processus de coupures homolytiques consécutives. Les principaux exemples connus de l'homme du métier sont les esters d'oxime de benzoyle, les arylsulfides, les peroxydes, les peroxydes contenant un chromophore tel que la benzophénone ou toute alkylphénone, les disulfides, les cétosulfides et les composés azoïques comme l'AIBN (azobisisobutyronitrile) et les azobenzoines.

Selon une seconde variante de cet aspect, le photoamorçeur est du type apte à créer des radicaux libres par un mécanisme d'arrachement d'atome. La classe la plus courante de ces photoamorçeurs est celle dans laquelle un proton est arraché à un substrat au cours de la photoréduction d'un état triplet nπ* du photoamorçeur, les principaux exemples étant les dérivés de la benzophénone, des thioxanthones, du benzyle, des 1,2- dicétones, telles que la camphorquinone, et des cétocoumarines. Le formulateur dispose d'autres composés photosensibles, les sels d'onium tels que notamment les sels de triarylsulfonium, les sels d'alkylarylsulfonium et les sels de diarylhalonium. Ces composés sont généralement très employés comme photoamorçeur de polymérisation cationique, cependant l'interaction de l'état triplet avec un donneur de proton produit des radicaux libres permettant d'amorcer également un processus radicalaire.

Selon une troisième variante de cet aspect, le photoamorçeur est du type photoréductible. La création de radicaux libres est consécutive à un transfert d'électron. Les familles de composés fonctionnant ainsi sont des chromophores de type diarylcétone, camphorquinone, cétocoumarine ou des colorants aromatiques du type xanthène, fluorone, thioxanthone, thiazine, acridine, anrthraquinone, cyanine, mérocyanine, benzopyrane. La famille des colorants aromatiques photoréductibles est tout particulièrement susceptible d'intéresser le formulateur de résines dentaires dans la mesure où ces composés aromatiques photoréductibles sont excitables dans des domaines de longueur d'onde du visible.

Selon un second aspect des moyens d'amorçage, le système d'amorçage peut être un système bicomposant (photoamorçeur + coamorçeur). Dans le cadre de la présente invention, la description de cet aspect est limitée au cas de la polymérisation radicalaire en chaine dans lequel les deux composants réalisent entre eux un transfert d'électron sous irradiation à la longueur d'onde λ. Cela n'est qu'un cas particulier du concept de système de photoamorcage bicomposant.

Une possibilité est la combinaison d'une espèce photosensible accepteur d'électron et d'une espèce donneur d'électron. Cette combinaison est généralement la plus décrite et permet d'envisager de nombreuses associations. Ainsi, les espèces photosensibles accepteur d'électron recoupent l'ensemble des familles précédemment exposées à propos des photoamorçeurs photoréductibles. Dans cette stratégie, l'espèce donneur d'électron accélère la polymérisation radicalaire au cours d'un processus en deux étapes, à savoir un transfert d'électron suivi d'un transfert de proton. Il existe de nombreuses espèces susceptibles d'interagir avec les espèces photoréductibles et l'on peut citer principalement les composés comprenant un atome d'azote activé tels que la triéthanolamine, une amine tertiaire ou une arylamine tertiaire, en particulier la N,N-diméthyl-p-toluidine, la N,N-diéthanol-p-toluidine, la N,N-diméthyl-sym(m)xylidine, la 3,5-di-tert-butylaniline ou la N,N-diméthyl-p-aminobenzoate d'éthyle, les composés comprenant un atome d'azote et un atome de souffre activés tels que les dérivés thiazole, en particulier le mercaptobenzothiazole, les sels de bore (borate) tels que les dérivés de tetraphénylborate et, de manière plus préférée, les dérivés de butyltriphénylborate. En raison d'une forte absorption en dessous d'une longueur d'onde de 300 nm et d'une « queue » du spectre dans le visible au delà d'une longueur d'onde de 400 nm, voire 430 nm dans certains cas, ces composés sont photosensibles tant dans le domaine UV que dans le domaine du visible.

Selon un troisième aspect des moyens d'amorçage, le système d'amorçage est un système de photoamorçage comprenant, en plus du photoamorçeur à proprement parler, au moins une espèce, dénommée photosensibilisateur, apte à photosensibiliser le ou les moyens d'amorçage créant les centres actifs (radicaux libres). Dans ce cas, l'interaction entre le photosensibilisateur dans l'un de ses états excités avec le photoamorçeur peut, principalement et sans caractère exhaustif de la description, être de deux natures. La photosensibilisation opère soit par transfert d'énergie état singulet-état singulet ou état triplet-état triplet, soit par transfert d'électron photo-induit avec le photosensibilisateur, soit par les deux à la fois.

Si l'un au moins des photoamorçeurs est du type apte à générer des radicaux libres par un mécanisme de photoclivage homolytique, alors il existe un certain nombre de systèmes décrits dans la littérature qui permettent d'accélérer ce processus. Ainsi une classe importante de ces amorceurs est celle des peroxydes, mais les mécanismes de photosensibilisation sont très variés. Par exemple, le peroxyde de benzoyle, le peroxyde de décanoyle peuvent être photosensibilisés par un transfert d'énergie depuis l'état triplet de composés tels que l'anthracène, l'acétophénone, les métoxy- et cyanobenzophénones, associés à la formation d'un complexe à transfert de charge. Dans d'autres cas, il s'agit d'un transfert d'électron comme par exemple entre un thioxanthène et la 3,3',4,4'-tétra-(t-butylperoxycarbonyl)-1-benzophénone. Ces exemples ne sont fournis qu'à titre indicatif, compte tenu de la variabilité des mécanismes de sensibilisation en fonction des conditions opératoires et des espèces chimiques en jeu.

D'autres exemples d'importance concernent l'interaction qui existe entre une α-amino acétophénone telle que la α-morpholino thiométhylphénylcétone, et un photosensibilisateur de type thioxanthone. Ce système a la particularité de pouvoir fonctionner tant par un transfert d'énergie depuis l'état triplet de la thioxanthone que par un transfert d'électron.

Il est par ailleurs possible de photosensibiliser certains des systèmes bicomposants qui associent un donneur et un accepteur d'électron. En particulier, le triptyque sel d'onium, sensibilisateur «intermédiaire» photoréductible et donneur d'électron, est très efficace en amorçage de formulations acryliques. On peut notamment réaliser les combinaisons suivantes entre :
- Un sel d'onium choisi parmi les sels de diaryliodonium et les sels de triarylsulfonium,
- Une espèce photosensible et photoréductible intermédiaire de type di-aminoarylcétone, cétocoumarine, thioxanthone, xanthène, fluorone, thiazine, acridine, anthraquinone, cyanine, mérocyanine et benzopyrane et leurs dérivés, et
- Un donneur d'électrons tel que les composés comprenant un atome d'azote activé, comme par exemple une amine tertiaire et une arylamine tertiaire, en particulier la N,N-diméthyl-p-toluidine, la N,N-diéthanol p-toluidine, la N,N-diméthyl-sym(m)xylidine, la 3,5-di-tert-butylaniline ou la N,N-diméthyl p-aminobenzoate d'éthyle, les composés comprenant un atome d'azote et un atome de soufre activés tels que les dérivés de thiazole, en particulier le mercaptobenzothiazole, les sels de bore (borate) tels que, par exemple le tétraphénylborates ou les butyltriphénylborates, et autres sels de type tétraorganylborates de tétraalkylammonium.

On pourra considérer différemment le système suivant que l'on considère l'espèce «intermédiaire» ou le sel d'onium comme le photosensibilisateur.

Par ailleurs, une autre série d'exemples applicables dans le cadre de l'invention est relative à la photosensibilisation d'un sel d'onium, du type sel de triarylsulfonium ou sel de diarylhalogénium utilisé sans association, avec un donneur d'électron du type amine tertiaire ou sel de borate, éventuellement associé à un donneur de proton approprié.

Le formulateur peut alors éventuellement choisir de photosensibilisateur par addition d'un composé photosensible permettant un transfert d'énergie état triplet-état triplet choisi parmi l'acétone, la 1-indone, l'acétophénone, la 3-trifluorométhyl-acétophénone et la xanthone, ou en incorporant un composé photosensible permettant un transfert d'électron avec un sel d'onium choisi parmi l'anthracène, le pyrène, le pérylène, les cétones aromatiques telle que la benzophénone, les cétones de Michler, les xanthones, les thioxanthones, les dérivés de diméthylaminobenzylidine, les phénanthraquinones, l'éosine, les cétocoumarines, les acridines et les benzofuranes.

Selon un dernier aspect des moyens d'amorçage de la réaction de polymérisation, ceux-ci sont des moyens d'amorçage de type photochimique, dont la composition peut comprendre au moins un photoamorçeur, au moins un coamorceur, au moins un photosensibilisateur ou, plus généralement, toute combinaison adéquate entre les différents composants potentiels d'un système de photoamorçage, tels que décrits précédemment, dans le but d'augmenter son efficacité et/ou de modifier son domaine d'absorption du rayonnement électromagnétique.

Dans la composition selon l'invention, ledit moyen d'amorçage photochimique de polymérisation est de préférence choisi parmi la camphorquinone, l'éthyl-4-diméthylaminobenzoate, l'oxide de bisacylphosphine, l'oxyde de 2,4,6-triméthylbenzoyldiphenylphosphine, et le système camphorquinone/éthyl-4-diméthylaminobenzoate.

Selon l'invention, le système de suivi de polymérisation est un système visuel par changement de couleur d'un indicateur coloré contenu dans la composition. Cet indicateur permet de signaler un changement du milieu, par exemple la variation du pH, de la réaction d'oxydoréduction ou d'un changement de phase. Dans notre cas, la réaction de polymérisation induit un changement de milieu, c'est-à-dire un changement de potentiel dans le cas d'indicateur d'oxydoréduction ou de pH dans le cas d'un indicateur de pH, qui est mis en évidence par le virage de l'indicateur coloré.

Les compositions selon l'invention comportent au moins un indicateur coloré qui pourra être choisi aisément par l'homme du métier.

En particulier, ledit indicateur coloré pourra être choisi parmi le bleu de bromophénol, le bleu de bromothymol, le bleu de méthylène, et le rouge de méthyle.

Selon un mode de réalisation, la composition de l'invention peut comprendre en outre un filtre anti-UV, de préférence du type de ceux utilisé en cosmétique pour absorber les UVA.

Selon une variante de réalisation, ladite composition peut comprendre en outre jusqu'à 5 % dudit filtre anti-UV.

En particulier, ledit filtre anti-UV peut être choisi parmi l'éthyl-4-aminobenzoate éthoxylé, le 3-benzylidène camphre, et leurs mélanges.

Un second objet de la présente invention porte sur un procédé pour faire une barrière dentaire, caractérisé en ce qu'il comprend les étapes suivantes :
a) l'application sur les tissus dentaires à protéger, en particulier les tissus gingivaux, à l'aide d'un embout applicateur, d'une composition telle que définie précédemment,
b) l'irradiation par une lampe à photo-polymériser au niveau de la zone des tissus dentaires recouverte de ladite composition, jusqu'à observation d'un changement de couleur de la composition indiquant le caractère complet de la polymérisation.

L'invention concerne ainsi l'utilisation d'une composition selon l'invention pour obtenir une barrière dentaire, qui est générée *in situ* sur l'endroit d'application de ladite composition.

Un troisième objet de la présente invention porte sur une barrière dentaire caractérisée en ce qu'elle est obtenue selon le procédé tel que défini précédemment ou à partir d'une composition précurseur telle que définie précédemment.

Les barrières dentaires obtenues selon l'invention présentent des propriétés d'adhésion avantageuses de la barrière dentaire aux tissus dentaires sur lequel elle est appliquée, en particulier les tissus gingivaux, permettant une protection optimale de la zone de la bouche à protéger.

Par ailleurs, les barrières dentaires obtenues selon l'invention présentent une grande simplicité d'utilisation, car elles peuvent être facilement retirées de la gencive à l'aide d'une pince à la fin du traitement.

Un dernier objet de la présente invention porte sur l'utilisation d'une barrière dentaire telle que définie précédemment, caractérisée en ce qu'elle permet de protéger les tissus dentaires, en particulier la gencive, notamment lors d'un traitement au cabinet dentaire, en particulier un traitement de blanchiment au fauteuil.

En particulier, selon l'invention, d'une part, ladite barrière permet d'isoler électriquement les tissus dentaires, en particulier la gencive, lors d'un traitement au cabinet dentaire, en particulier un traitement de blanchiment au fauteuil.
On peut citer par exemple le traitement de blanchiment électrophorétique décrit dans le brevet FR 2 844 719. La barrière est alors utilisée comme protection gingivale ayant pour fonction l'isolement de la gencive par rapport à la dent afin d'assurer le passage du courant au travers de la dent.

En particulier, selon l'invention, d'autre part, ladite barrière permet d'isoler chimiquement les tissus dentaires, en particulier la gencive, notamment lors d'un traitement au cabinet dentaire, en particulier un traitement de blanchiment au fauteuil.
En effet, un traitement de blanchiment des dents utilise généralement de fortes concentrations de peroxyde d'hydrogène qui est un puissant oxydant pouvant occasionner des brûlures.

Par ailleurs, en particulier, selon l'invention, ladite barrière permet d'isoler thermiquement les tissus dentaires, en particulier la gencive, notamment lors d'un traitement au cabinet dentaire, en particulier un traitement de blanchiment au fauteuil.
En effet, un traitement de blanchiment des dents utilise généralement une forte source lumineuse susceptible de générer une chaleur importante.

En outre, selon l'invention, ladite barrière permet d'isoler les tissus dentaires, en particulier la gencive, par rapport à l'exposition aux UVA, qui peuvent être nocif lors d'une exposition prolongée, notamment lors d'un traitement au cabinet dentaire, en particulier un traitement de blanchiment au fauteuil.

D'autres buts, caractéristiques et avantages de l'invention apparaîtront clairement à la lumière de la description explicative qui va suivre faite en référence aux divers exemples de réalisation de l'invention donnés simplement à titre d'illustration et qui ne sauraient donc en aucune façon limiter la portée de l'invention. Dans les exemples, tous les pourcentages sont donnés en poids, la température est en degrés Celsius, la pression est la pression atmosphérique, sauf indication contraire.

### DESCRIPTION DES FIGURES

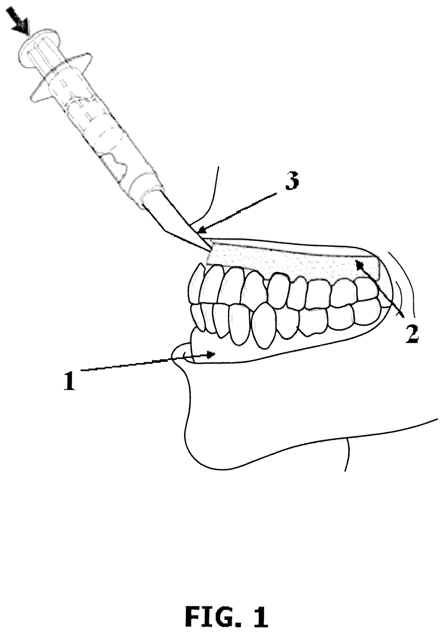

La figure 1 présente le mode d'application de la composition selon l'invention (2) sur une gencive (1) à l'aide d'un embout applicateur (3).

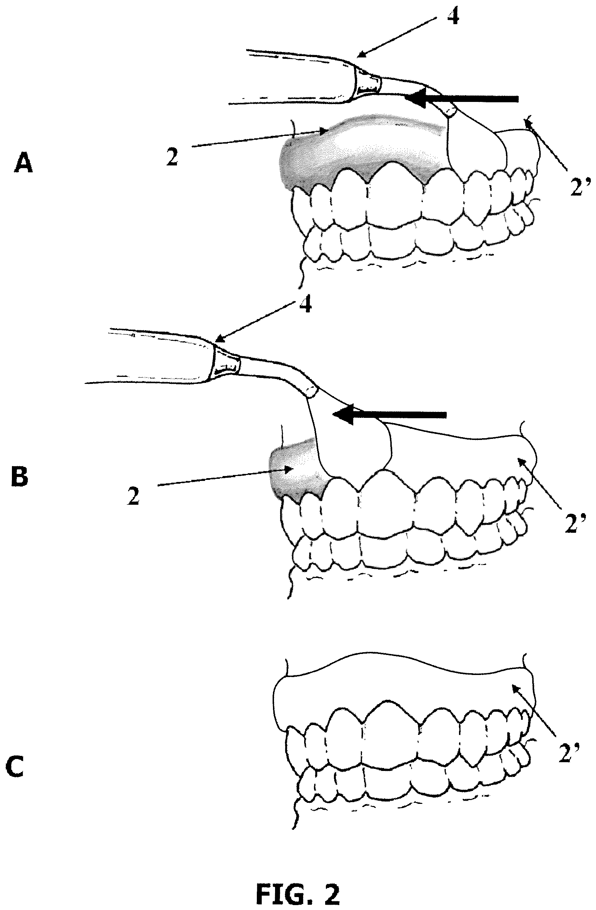

La figure 2 présente chronologiquement la polymérisation de la digue dentaire (2') à partir de la composition (2) qui est polymérisée par irradiation de la lampe à photopolymériser (4). On note un changement de couleur entre la composition polymérisable (2) et la composition polymérisée (2'), due à la présence dans la composition d'un indicateur coloré suivant la réaction de polymérisation.

Sur les images A et B, on voit la progression de la polymérisation à l'aide d'une lampe à photopolymériser (4), de la composition selon l'invention (2), qui se transforme peu à peu en une barrière dentaire (2').

Sur l'image C, qui est réalisée après polymérisation totale de la composition (2), on voit la barrière dentaire (2') obtenue, recouvrant parfaitement la gencive.

### EXEMPLES

### MATERIEL ET METHODES

### Matériel utilisé

La lampe à photopolymériser est une lampe à usage dentaire de type Mini L.E.D. SATELEC ayant une puissance maximum à une longueur d'onde de 450 nm et une intensité lumineuse significative pour des longueurs d'onde comprises entre 420 et 480 nm. La densité de puissance optique maximum de la lampe est de 1100 mW/cm².

### Méthodes de préparation d'une composition selon l'invention

Les méthodes employées pour préparer les compositions selon l'invention sont des méthodes bien connues de l'homme du métier. Nous ne citons ici que quelques exemples particuliers.

### - Synthèse de la composition A selon l'invention :

On mélange le diuréthane méthacrylate, le triéthylène glycol diméthacrylate (le ou les monomères) et le bleu de méthylène (le colorant), puis le mélange est agité pendant 5 heures à l'aide d'un agitateur magnétique jusqu'à dissolution complète du colorant.

On ajoute lentement sous agitation la silice colloïdale déshydratée (épaississant) jusqu'à homogénéisation du mélange. Enfin, on ajoute à l'abri de la lumière l'ethyl-4-diméthylaminobenzoate et la camphorquinone (système d'amorçage) puis le mélange est homogénéisé sous agitation.

### - Synthèse de la composition E selon l'invention :

On mélange le diuréthane diméthacrylate, le triéthylène glycol diméthacrylate (le ou les monomères) et le bleu de bromophénol (le colorant), puis le mélange est agité pendant 30 minutes à l'aide d'un agitateur magnétique jusqu'à dissolution complète du colorant.

Le mélange est alors chauffé à 60°C et placé sous agitation puis le Compritol 888® (épaisissant) est incorporé progressivement au mélange jusqu'à ce qu'il fonde et forme une solution homogène.

Le mélange est alors refroidi à température ambiante.

Enfin, l'ethyl-4-diméthylaminobenzoate et la camphorquinone (système d'amorçage) sont ajoutés à l'abri de la lumière puis le mélange est homogénéisé sous agitation.

### Méthode de mise en oeuvre de la composition pour former une barrière dentaire selon l'invention

La composition selon l'invention peut être appliquée sur une gencive à l'aide d'un embout applicateur, de type caoutchouteux. Puis le praticien peut utiliser une canule pour faire descendre le produit entre les dents de manière à bien couvrir l'ensemble de la gencive à protéger. Le praticien peut également utiliser directement et uniquement une canule pour appliquer la composition.

Une fois la composition polymérisable appliquée, on en réalise la polymérisation à l'aide d'une lampe à photopolymériser qui est appliquée durant quelques secondes au-dessus de la composition à polymériser. L'irradiation permet l'activation de la réaction de polymérisation, et ainsi la formation de la barrière dentaire. On note également un changement de couleur entre la composition polymérisable et la barrière dentaire obtenue, due à la présence dans la composition d'un indicateur coloré suivant la réaction de polymérisation.

### EXEMPLES DE COMPOSITIONS

### Exemple 1 : composition A selon l'invention

On prépare une composition comprenant :

| Produit | % massique |
|---|---|
| Diuréthane diméthacrylate | 42.0% |
| Triéthylène glycol diméthacrylate | 42.0% |
| Camphoroquinone | 0.4% |
| Ethyl-4-diméthylaminobenzoate | 0.4% |
| Aerosil 200® | 15.1% |
| Bleu de méthylène | 0.1% |

Le diuréthane diméthacrylate et le triéthylène glycol diméthacrylate sont ici les monomères qui constituent le copolymère.

La combinaison de la camphorquinone et de l'éthyl-4-diméthylaminobenzoate permet d'initier la polymérisation après exposition à une lampe dentaire émettant de la lumière bleue de longueur d'onde comprise entre 420 et 480 nm. Cette polymérisation est responsable de l'adhésion à un substrat organique comme par exemple un tissu buccal.

L'éthyl-4-diméthylaminobenzoate permet également d'absorber les UV-A.

L'Aérosil 200®' qui est une silice colloïdale anhydre, est utilisé dans la présente invention afin d'épaissir le milieu et favorisé sa stabilité au repos de par sa thixotropie.

Le bleu de méthylène permet de signaler la polymérisation par son changement de couleur.

### Exemple 2 : composition B selon l'invention

On prépare une composition comprenant :

| Produit | % massique |
|---|---|
| Diuréthane diméthacrylate | 59.0% |
| Triéthylène glycol diméthacrylate | 25% |
| Camphoroquinone | 0.4% |
| Ethyl-4-diméthylaminobenzoate | 0.4% |
| Aerosil 200® | 15.1% |
| Bleu de bromophenol | 0.1% |

Mêmes remarques que pour la composition A selon l'invention.

Ici, le bleu de bromophénol permet de signaler la polymérisation par son changement de couleur.

### Exemple 3 : composition C selon l'invention

On prépare une composition comprenant :

| Produit | % massique |
|---|---|
| bisphénol A diméthacrylate | 54.0% |
| Triéthylène glycol diméthacrylate | 30.0% |
| Lucirin® TPO | 0.8% |
| Ethyl-4-aminobenzoate éthoxylé | 5.0% |
| Aerosil 400® | 10.0% |
| Rouge de méthyle | 0.2% |

Le bisphénol A diméthacrylate et le triéthylène glycol diméthacrylate sont ici les monomères qui constituent le copolymère.

La Lucirin® TPO, qui est l'oxyde de 2,4,6-Trimethylbenzoyldiphenylphosphine, permet d'initier la polymérisation après exposition à une lampe dentaire émettant de la lumière bleue de longueur d'onde comprise entre 420 et 460 nm. Cette polymérisation est responsable de l'adhésion à un substrat organique comme par exemple un tissu buccal.

L'éthyl-4-aminobenzoate éthoxylé permet d'absorber les UV-A.

L'Aérosil 400®, qui est une silice colloïdale, est utilisé dans la présente invention afin d'épaissir le milieu.

Le rouge de méthyle permet de signaler la polymérisation par son changement de couleur.

### Exemple 4 : composition D

On prépare une composition comprenant :

| Produit | % massique |
|---|---|
| Méthacrylate de méthyle | 40.0% |
| Uréthane diméthacrylate | 44.0% |
| Oxyde de bisacylphosphine | 1.4% |
| 3-benzylidène camphre | 1.5% |
| Aerosil 200® | 13.0% |
| Bleu de méthylène | 0.1% |

Le méthacrylate de méthyle et l'uréthane diméthacrylate sont ici les monomères qui constituent le copolymère.

L'oxyde de bisacylphophine permet d'initier la polymérisation après exposition à une lampe dentaire émettant de la lumière bleue de longueur d'onde comprise entre 400 et 440 nm. Cette polymérisation est responsable de l'adhésion à un substrat organique comme par exemple un tissu buccal.

Le 3-benzylidène camphre permet d'absorber les UV-A.

L'Aérosil 200® dans la présente invention est utilisé afin d'épaissir le milieu.

Le bleu de méthylène permet de signaler la polymérisation par son changement de couleur.

### Exemple 5 : composition E selon l'invention

On prépare une composition comprenant :

| Produit | % massique |
|---|---|
| Diuréthane diméthacrylate | 40.0% |
| Triéthylène glycol diméthacrylate | 39.6% |
| Camphoroquinone | 0.4% |
| Ethyl-4-diméthylaminobenzoate | 0.4% |
| Compritol 888® | 19.1% |
| Bleu de bromophénol | 0.5% |

Mêmes remarques que pour les exemples 1 et 2.

Le Compritol 888®, qui est un dibéhennate de glycérol, est utilisé dans la présente invention afin d'épaissir le milieu et favorisé sa stabilité au repos.

## Revendications

1. Composition précurseur pour barrière dentaire, comprenant :
- un mélange de monomères choisis parmi le diuréthane diméthacrylate, le triéthylène glycol diméthacrylate, le bisphénol A diméthacrylate, et le méthacrylate de méthyle, ledit mélange de monomères représentant 50 à 90 % en poids de la composition ;
- au moins un épaississant, représentant 10 à 49 % en poids de la composition ;
- un moyen d'amorçage photochimique de polymérisation, représentant jusqu'à 2 % en poids de ladite composition ; et
- au moins un indicateur coloré pour suivre la réaction de polymérisation, représentant jusqu'à 5 % en poids de la composition.

2. Composition selon la revendication 1, **caractérisée en ce que** ledit mélange est constitué du diuréthane diméthacrylate et du triéthylène glycol diméthacrylate.

3. Composition selon l'une des revendications 1 à 2, **caractérisée en ce que** ledit au moins un épaississant est choisi parmi l'alcool cétylique, la silice colloïdale anhydre, le dibéhénate de glycérol, l'acide alginique, l'alginate de sodium, l'alginate de potassium, l'alginate d'ammonium, l'alginate de calcium, l'alginate de propane-1,2-diol, l'agar, la carragénanne, le furcelleran, la gomme de caroube, la gomme d'avoine, la gomme de Guar, le tragacanthe, la gomme arabique, la gomme xanthane, la gomme karaya, la gomme gellane, le sorbitol, le mannitol, le glycerol, le stéarate de polyoxyéthylène, le stéarate de polyoxyéthylène, le monolaurate de polyoxyéthylène-20-sorbitan, le monooléate de polyoxyéthylène-20-sorbitan, le monopalmitate de polyoxyéthylène-20-sorbitan, le monostearate de polyoxyéthylène-20-sorbitan, le tristéarate de polyoxyéthylène-20-sorbitan, la pectine, la gélatine, la cellulose, la cellulose de méthyle, la cellulose d'éthyle, la cellulose d'hydroxypropyle, la cellulose d'hydroxypropylméthyle, la cellulose de méthyléthyle, la cellulose de carboxyméthyl, les sels d'acide gras, les acides gras mono- and di-glycérides, les esters de mono- and di-glycérides, les sucre d'esters d'acides gras, les sucre glycérides, les esters polyglycérol d'acides gras, le polyricinoléate polyglycérol, les esters propylèneglycol d'acides gras, les mélange d'esters glycérol- and propylèneglycol d'acide lactique et d'acides gras, l'huile de soja estérifiée, le stéarate de sorbitane, le tristéarate de sorbitane, le monolaurate de sorbitane, le monooléate de sorbitane, le monopalmitate de sorbitane et leurs mélanges.

4. Composition selon l'une des revendications 1 à 3, **caractérisée en ce que** ledit moyen d'amorçage photochimique de polymérisation est choisi parmi la camphorquinone, l'éthyl-4-diméthylaminobenzoate, l'oxide de bisacylphosphine, l'oxyde de 2,4,6-triméthylbenzoyldiphenylphosphine, et le système camphorquinone/éthyl-4-diméthylaminobenzoate.

5. Composition selon l'une des revendications 1 à 4, **caractérisée en ce que** ledit au moins un indicateur coloré est choisi parmi le bleu de bromophénol, le bleu de méthylène, bleu de bromothymol et le rouge de méthyle.

6. Composition selon l'une des revendications 1 à 5, **caractérisée en ce qu'**elle comprend en outre un filtre anti-UV, de préférence du type de ceux utilisé en cosmétique pour absorber les UVA.

7. Composition selon la revendication 6, **caractérisée en ce qu'**elle comprend en outre jusqu'à 5 % dudit filtre anti-UV.

8. Composition selon la revendication 6 ou 7, **caractérisée en ce que** ledit filtre anti-UV est choisi parmi l'éthyl-4-aminobenzoate éthoxylé, le 3-benzylidène camphre, et leurs mélanges.

9. Procédé pour faire une barrière dentaire, **caractérisé en ce qu'**il comprend les étapes suivantes :
a) l'application sur les tissus dentaires à protéger, en particulier les tissus gingivaux, à l'aide d'un embout applicateur, d'une composition selon l'une des revendications 1 à 8,
b) l'irradiation par une lampe à photo-polymériser au niveau de la zone des tissus dentaires recouverte de ladite composition, jusqu'à observation d'un changement de couleur de la composition indiquant le caractère complet de la polymérisation.

10. Barrière dentaire **caractérisée en ce qu'**elle est obtenue selon le procédé de la revendication 9 ou à partir de la composition précurseur selon l'une quelconque des revendications 1 à 8.

11. Utilisation d'une barrière dentaire selon la revendication 10, pour protéger les tissus dentaires, en particulier la gencive, notamment lors d'un traitement au cabinet dentaire, en particulier un traitement de blanchiment au fauteuil.

12. Utilisation d'une barrière dentaire selon la revendication 11, pour isoler électriquement et/ou isoler chimiquement et/ou isoler thermiquement et/ou isoler des UVA lesdits tissus dentaires.

## Patentansprüche

1. Vorläuferzusammensetzung für einen Zahnschutz, umfassend:
- ein Gemisch von Monomeren, ausgewählt aus Diurethandimethacrylat, Triethylenglykoldimethacrylat, Bisphenol-A-Dimethacrylat, und Methylmethacrylat, wobei das Gemisch von Monomeren 50 bis 90 Gew.-% der Zusammensetzung darstellt,
- mindestens ein Verdickungsmittel, das 10 bis 49 Gew.-% der Zusammensetzung darstellt,
- ein Mittel zur photochemischen Erregung einer Polymerisation, das bis zu 2 Gew.-% der Zusammensetzung darstellt, und
- mindestens einen Farbindikator zum Verfolgen der Polymerisationsreaktion, der bis zu 5 Gew.-% der Zusammensetzung darstellt.

2. Zusammensetzung nach Anspruch 1, **dadurch gekennzeichnet, dass** das Gemisch aus Diurethandimethacrylat und Triethylenglykoldimethacrylat gebildet wird.

3. Zusammensetzung nach einem der Ansprüche 1 bis 2, **dadurch gekennzeichnet, dass** das mindestens eine Verdickungsmittel ausgewählt ist aus Cetylalkohol, wasserfreier kolloidaler Kieselsäure, Glyceroldibehenat, Alginsäure, Natriumalginat, Kaliumalginat, Ammoniumalginat, Calciumalginat, Propan-1,2-diol-Alginat, Agar-Agar, Carrageen, Furcellaran, Johannisbrotkernmehl, Hafergummi, Guargummi, Traganth, Gummi arabicum, Xanthangummi, Karayagummi, Gellangummi, Sorbitol, Mannitol, Glycerol, Polyoxyethylenstearat, Polyoxyethylen-20-Sorbitanmonolaurat, Polyoxyethylen-20-Sorbitanmonooleat, Polyoxyethylen-20-Sorbitanmonopalmitat, Polyoxyethylen-20-Sorbitanmonostearat, Polyoxyethylen-20-Sorbitantristearat, Pektin, Gelatine, Cellulose, Methylcellulose, Ethylcellulose, Hydroxypropylcellulose, Hydroxypropylmethylcellulose, Methylethylcellulose, Carboxymethylcellulose, Fettsäuresalzen, Fettsäuremono- und -diglyceriden, Mono- und Diglyceridestern, Zuckern von Fettsäureestern, Zuckerglyceriden, Fettsäurepolyglycerinestern, Polyglycerin-Polyricinoleat, Fettsäurepropylenglykolestern, Gemischen von Glycerinestern und Propylenglykolmilchsäure und Fettsäuren, verestertem Sojaöl, Sorbitanstearat, Sorbitantristearat, Sorbitanmonolaurat, Sorbitanmonooleat, Sorbitanmonopalmitat und ihren Gemischen.

4. Zusammensetzung nach einem der Ansprüche 1 bis 3, **dadurch gekennzeichnet, dass** das Mittel zur photochemischen Erregung der Polymerisation ausgewählt ist aus Campherchinon, Ethyl-4-dimethylaminobenzoat, Bisacylphosphinoxid, 2,4,6-Trimethylbenzoyldiphenylphosphinoxid und dem System Campherchinon/Ethyl-4-dimethylaminobenzoat.

5. Zusammensetzung nach einem der Ansprüche 1 bis 4, **dadurch gekennzeichnet, dass** der mindestens eine Farbindikator ausgewählt ist aus Bromphenolblau, Methylenblau, Bromthymolblau und Methylrot.

6. Zusammensetzung nach einem der Ansprüche 1 bis 5, **dadurch gekennzeichnet, dass** sie ferner einen Anti-UV-Filter, vorzugsweise von dem Typ umfasst, der in der Kosmetik zur Absorption von UVA-Strahlung verwendet wird.

7. Zusammensetzung nach Anspruch 6, **dadurch gekennzeichnet, dass** sie ferner bis zu 5 % des Anti-UV-Filters umfasst.

8. Zusammensetzung nach Anspruch 6 oder 7, **dadurch gekennzeichnet, dass** der Anti-UV-Filter ausgewählt ist aus ethoxyliertem Ethyl-4-aminobenzoat, 3-Benzylidencampher und ihren Gemischen.

9. Verfahren zur Herstellung eines Zahnschutzes, **dadurch gekennzeichnet, dass** es die folgenden Schritte umfasst:
a) Applikation auf zu schützendes Zahngewebe, insbesondere das Zahnfleischgewebe, einer Zusammensetzung nach einem der Ansprüche 1 bis 8 mithilfe eines Applikatoraufsatzes,
b) Bestrahlung durch eine Photopolymerisationslampe in Bezug auf den Bereich des Zahngewebes, der von der Zusammensetzung bedeckt ist, bis eine Farbveränderung der Zusammensetzung zu beobachten ist, welche die Vollständigkeit der Polymerisation anzeigt.

10. Zahnschutz, **dadurch gekennzeichnet, dass** er gemäß dem Verfahren nach Anspruch 9 oder aus der Vorläuferzusammensetzung nach einem der Ansprüche 1 bis 8 erhalten wird.

11. Verwendung eines Zahnschutzes nach Anspruch 10 zum Schutz des Zahngewebes, insbesondere des Zahnfleischs, besonders während einer zahnärztlichen Behandlung, insbesondere eines Bleachings auf dem Behandlungsstuhl.

12. Verwendung eines Zahnschutzes nach Anspruch 11 zum elektrischen Isolieren und/oder chemischen Isolieren und/oder thermischen Isolieren und/oder Isolieren von UVA-Strahlung des Zahngewebes.

## Claims

1. A precursor composition for a dental barrier, comprising:
- a mixture of monomers selected from diurethane dimethacrylate, triethylene glycol dimethacrylate, bisphenol A dimethacrylate and methyl methacrylate, said mixture of monomers making up from 50 to 90 weight % of the composition;
- at least one thickener making up from 10 to 49 weight % of the composition;
- a photochemical means of initiating polymerization making up to 2 weight % of said composition; and
- at least one colored indicator for monitoring the polymerization reaction making up to 5 weight % of the composition.

2. The composition of claim 1, wherein said mixture consists of diurethane dimethacrylate and triethylene glycol dimethacrylate.

3. The composition of any of claims 1 to 2, wherein said at least one thickener is selected from cetyl alcohol, anhydrous colloidal silica, glycerol dibehenate, alginic acid, sodium alginate, potassium alginate, ammonium alginate, calcium alginate, propane-1,2-diol alginate, agar, carrageenan, furcellaran, carob gum, oat gum, guar gum, tragacanth, gum arabic, xanthan gum, gum karaya, gellan gum, sorbitol, mannitol, glycerol, polyoxyethylene stearate, polyoxyethylene-20-sorbitan monolaurate, polyoxyethylene-20-sorbitan monooleate, polyoxyethylene-20-sorbitan monopalmitate, polyoxyethylene-20-sorbitan monostearate, polyoxyethylene-20-sorbitan tristearate, pectin, gelatin, cellulose, methyl cellulose, ethyl cellulose, hydroxypropyl cellulose, hydroxypropylmethyl cellulose, methylethyl cellulose, carboxymethyl cellulose, salts of fatty acids, mono- and diglyceride fatty acids, esters of mono- and diglycerides, sugars of esters of fatty acids, sugar glycerides, polyglycerol esters of fatty acids, polyglycerol polyricinoleate, propylene glycol esters of fatty acids, mixtures of glycerol and propylene glycol esters of lactic acid and of fatty acids, esterified soya oil, sorbitan stearate, sorbitan tristearate, sorbitan monolaurate, sorbitan monooleate, sorbitan monopalmitate and mixtures thereof.

4. The composition of any of claims 1 to 3, wherein said photochemical means of initiating polymerization is selected from camphorquinone, ethyl-4-dimethylaminobenzoate, bisacylphosphine oxide, 2,4,6-trimethylbenzoyldiphenylphosphine oxide and the camphorquinone/ethyl-4-dimethylaminobenzoate system.

5. The composition of any of claims 1 to 4, wherein said at least one colored indicator is selected from bromophenol blue, methylene blue, bromothymol blue and methyl red.

6. The composition of any of claims 1 to 5, which further comprises a UV filter, preferably of the type of those used in cosmetics to absorb UV-A.

7. The composition of claim 6, which comprises up to 5% of said UV filter.

8. The composition of claim 6 or claim 7, wherein said UV filter is selected from ethoxylated ethyl-4-aminobenzoate, 3-benzylidene camphor and mixtures thereof.

9. A method for making a dental barrier, which comprises the following steps:
a) applying a composition of any of claims 1 to 8 on the dental tissues to be protected, notably on the gingival tissues, by means of an applicator nozzle,
b) irradiating, with a photopolymerization lamp, the area of the dental tissues covered with said composition, until a color change of the composition is observed, indicating that polymerization has gone to completion.

10. A dental barrier, which is obtained by the method of claim 9 or from the precursor composition of any of claims 1 to 8.

11. Use of the dental barrier of claim 10 for protecting dental tissues, notably gingiva, during treatment in the dental office, notably an office-bleaching treatment wherein the dental barrier of claim 14 is applied on said dental tissues for protecting them.

12. The use of the dental barrier of claim 11, for electrically and/or chemically and/or thermally isolating said dental tissues from UV-A.
